Europäisches Patentamt

European Patent Office

Office européen des brevets

(11) Veröffentlichungsnummer: **0 399 248**
**A2**

# (12) EUROPÄISCHE PATENTANMELDUNG

(21) Anmeldenummer: **90108214.9**

(22) Anmeldetag: **30.04.90**

(51) Int. Cl.5: **A61F 13/02, A61L 15/44**

(30) Priorität: **25.05.89 CH 1971/89**

(43) Veröffentlichungstag der Anmeldung:
**28.11.90 Patentblatt 90/48**

(84) Benannte Vertragsstaaten:
**AT BE CH DE DK ES FR GB GR IT LI LU NL SE**

(71) Anmelder: **LTS LOHMANN
THERAPIE-SYSTEME GmbH & CO.KG
Irlicher Strasse 55
D-5450 Neuwied 12(DE)**

(72) Erfinder: **Eisenring, Martin
Innerfeld 40
CH 9606 Bütschwil(CH)**

(74) Vertreter: **Feldmann, Clarence Paul et al
c/o Patentanwaltsbüro FELDMANN AG
Postfach Kanalstrasse 17
CH-8152 Glattbrugg(CH)**

(54) **Heftpflaster.**

(57) In einem Heftpflaster ist zwischen der Deckschicht (4) und der oberen Schicht der Kompresse (5) ein Reservoir (4) eingebracht. Das Reservoir (4) kann somit entweder in der Kompresse (5) oder zwischen der Kompresse und der Deckschicht (1) liegen. Im Reservoir (4) ist ein Wirkstoff enthalten, der in einer flüssigen oder pastösen Substanz gelöst ist. Das Material des Reservoirs ist, für den Durchgang des Wirkstoffes und der Substanz in der sie gelöst ist, undurchlässig. Mittels Fingerdruck wird das Reservoir zerstört und der Wirkstoff tränkt die Kompresse.

FIG.1

## HEFTPFLASTER

Die vorliegende Erfindung betrifft ein Heftpflaster bestehend aus einer Deckschicht mit selbstklebenden Seitenstreifen, einer ein oder mehrlagigen Kompresse zur Wundauflage und einer abziehbaren Schutzschicht.

Solche Heftpflaster oder Schnellverbände sind seit vielen Jahren bekannt und in diversen Formen und Grössen auf dem Markt erhältlich.

Während vielen Jahren wurde an diesem prinzipiellen Aufbau nichts geändert. Die Erfahrungen haben zu zahlreichen Verbesserungen und Varianten der verschiedenen Materialien der einzelnen Schichten geführt. So sind Schnellverbände mit verschiedenartigen Deckschichten erhältlich. Auch der kompressenartige Bereich ist unterschiedlich gestaltet aus Gazen oder Vliese.

Erst in neuerer Zeit beschäftigt man sich auch mit Heft pflaster denen man bereits Wirkstoffe beigibt. Im nachfolgenden werden solche Heftpflaster als medizinische Pflaster bezeichnet. Teilweise werden solche Pflaster unter Weglassung der Kompresse gebildet, in dem eine wirkstoffhaltige Reservoirschicht angebracht ist. Solche Pflaster sind beispielsweise aus den EU-A-0 154 904, EU-A-0 154 904, EU-A-0 206 697, EU-A-0 148 391 sowie EU-A-0 204 968 bekannt. Sie zielen insbesondere auf eine Langzeitabgabe des Wirkstoffes ab, der in einer harzartigen Masse eingebettet ist. Meist handelt es sich bei den Wirkstoffen um solche zur transdermalen Applikation .

Für ähnliche Applikationen zeigt die CH-A-658 393 ein Pflaster bei dem anstelle der Kompresse ein Reservoir gebildet ist aus zwei unterschiedlichen Materialien, nämlich eine für den Durchgang des Wirkstoffes im wesentlichen undurchlässige Deckschicht und eine zur Hautauflage bestimmte Membran aus einem Material, welches die Abgabegeschwindigkeit des Wirkstoffes steuert. Das Reservoir ist somit ein Flüssigkeitsbehältnis mit nach Wahl der Membran steuerbarer Diffussionsgeschwindigkeit in dem ein rheologisches Mittel und Wirkstoff enthalten ist.

Im Gegensatz zu den vorherbeschriebenen medizinischen Pflaster, die auf eine Langzeitwirkung abzielen, werden bis heute Schnellverbände nicht mit einem Reservoir mit Wirkstoff ausgebildet. Zwar sind Heftpflaster bekannt mit Kompressen die mit einer Wirkstoff enthaltenden Lösung getränkt sind, die nachher dehydriert worden sind.

Soll zur Wundbehandlung ein in flüssiger oder pastöser Form vorliegender Wirkstoff aufgebracht werden, so muss dieses auch heute noch gesondert aufbewahrt, mitgenommen und zur gegeben Zeit direkt auf die Wunde aufgebracht werden und nachher durch das Heftpflaster abgedeckt werden.

Dies ist nicht nur umständlich, sondern oft auch nicht möglich, weil die erforderlichen Wirkstoffe nicht zur Hand sind.

Es ist die Aufgabe der vorliegenden Erfindung, ein Heftpflaster zu schaffen, welches aus einer Deckschicht mit selbstklebenden Seitenstreifen, einen ein oder mehrlagigen kompressenartigen Bereich zur Wundauflage und einer abziehbaren Schutzschicht besteht, welches zur sofortigen Abgabe von Wirkstoffen in flüssiger oder pastöser Form geeignet ist. Die vorliegende Aufgabe löst ein Heftpflaster mit den kennzeichnenden Merkmalen des Patentanspruches 1. Das erfindungsgemässe Heftpflaster ist, falls die abziehbare Schutzschicht nicht besonders gekennzeichnet ist, äusserlich kaum von einem herkömmlichen Pflaster unterscheidbar. In der angefügten Zeichnung ist daher in

Fig. 1 ein Schnitt durch eine beispielsweise Ausführung eines erfindungsgemässen Heftpflasters dargestellt;

Fig. 2 zeigt eine mögliche Ausführungsform mehrerer Reservoirs und

Fig. 3 einen Schnitt entlang der Linie $\overline{III} - \overline{III}$ in Fig. 2

Fig. 4 zeigte eine Variante entsprechend Figur 1. In

Fig. 5 ist die Schutzschicht des Pflasters sichtbar.

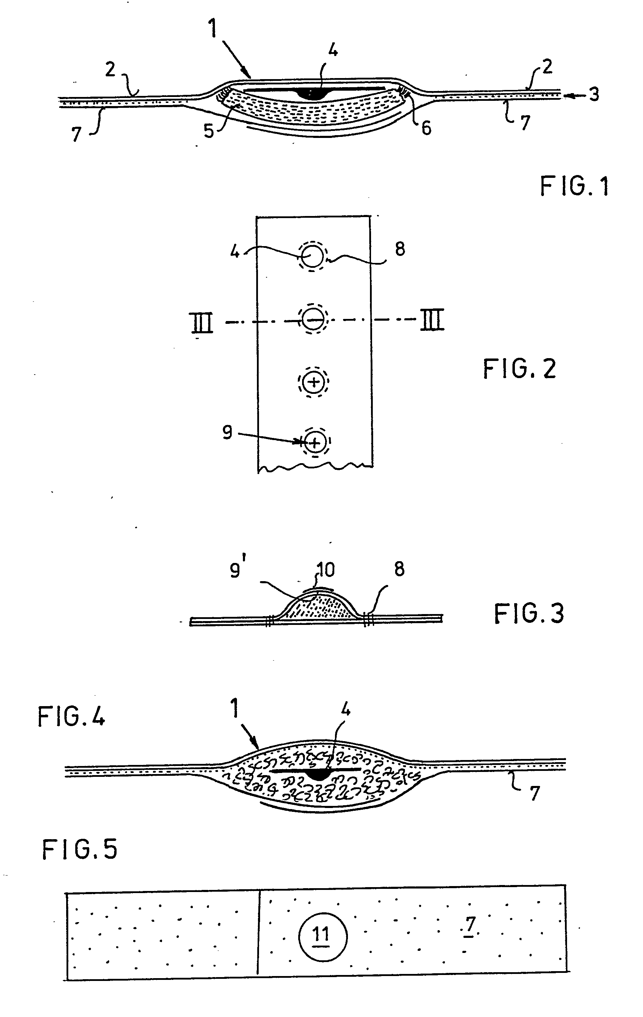

Die in der Figur 1 zuoberst gezeichnete Schicht ist die Deckschicht 1. Sie ist die eigentliche Trägerbahn an der alle anderen Teile direkt oder mittelbar haften. Die Deckschicht kann aus längs- und querelastischen textilen Flächengebilden oder einer atmungsaktiven Kunststoff-Folie bestehen. Dies ist für die Erfindung unwesentlich, genauso wie es auch unbedeutend ist, ob die Deckschicht hautfarbig oder transparent ist.

Entsprechend den heutigen Herstellungsverfahren wird auch das erfindungsgemässe Heftpflaster als Endlosbahn gefertigt und später nach Wunsch unterteilt. Die Seitenstreifen 2 der Deckschicht 1 sind mit einem geeigneten Klebstoff 3 beschichtet. Zwischen den beiden Seitenstreifen 2 ist in der Mitte ein in streifenform angeordnetes Reservoir 4 angebracht. Dieses wird von einer Kompresse 5 in Streifenform vollständig überdeckt. Die Kompresse 5 ist mit der Deckschicht 1 zwischen und angrenzend an die Seitenstreifen 2 mit der Deckschicht verbunden. Die Verbindung 6 zwischen der Deckschicht 1 und der Kompresse 5 kann nach bekannten Verfahren hergestellt sein. Je nach Art der Kompresse, ob diese aus einer Gaze oder einem Vlies besteht, kann die Verbindung durch kleben, schweissen oder nähen erfolgen. Abschliessend ist auf der Unterseite des Pflasters noch eine abziehbare Schutzschicht 7 vorgesehen, die mittels vom Klebstoff 3 gehalten wird. Die Schutzschicht 7 kann wie bisher aus zwei sich im Bereich der Kompresse 5 überlappenden Streifen hergestellt werden.

Die Figur 2 zeigt wie auch die Reservoire 4 sich wie das Heftpflaster in Form eines Endlosbandes herstellen lassen. Hierbei werden zwischen zwei Folien aus Kunststoff in regelmässigen Abständen Wirkstoffe in Lösung oder pastenförmiger Substanz durch Schweissnähte 8 eingeschlossen. Die entsprechenden Verfahren sind bekannt. Die so entstehenden kapselartigen Reservoire 4 können durch Fingerdruck zum Zerplatzen gebracht werden und die Kompresse mit ihrem Inhalt tränken. Sind die beiden Folien aus denen die Reservoirs hergestellt sind gleichstark, so ist nicht vorhersehbar, an welcher Stelle die Kapse zerstört wird. Ist die Deckschicht undurchlässig und der Wirkstoff in einer Flüssigkeit gelöst, so ist dies unwesentlich. In vielen Fällen ist jedoch eine gezielte Abgabe auf die Kompresse erwünscht und erforderlich. Dies lässt sich aber dadurch steuern, dass man in der zur Kompresse hin gerichteten Folie eine Sollzerstörungsstelle 9 anbringt. Hierzu kann eine Folie im Bereich der Sollzerstörungstelle 9 beispielsweise kreuzförmig angeritzt werden. In einem Versuchsaufbau wurden auch brauchbare Resultate erzielt, in dem der gelöste Stoff durch eine Folie hindurch injiziert wurde und die Einstichstelle 10 verklebt wurde. Die Injektionsstelle 9 wirkte nachher als Sollzerstörungsstelle.

Es ist auch denkbar, die Sollzerstörungsstelle durch punktuelle Bestrahlung, beispielsweise mit UV-Strahlen, zu bilden.

Die Variante nach Figur 4 ist gegenüber der Ausführung nach Figur 1 nur wenig geändert. Das Reservoir 4 ist hier als Kapsel dargestellt. Sie liegt nicht direkt an der Deckschicht 1 an, sondern ist zwischen zwei Schichten der Kompresse 5 und 4 aus Vliesstoff eingebettet.

In der Ausführung nach Figur 1 kann auch der Klebestoff auf die gesamte untere Seite der Deckschicht 1 aufgetragen sein, wodurch das Reservoir direkt auf dem Klebstoff der Deckschicht haftet und die das Rservoir überlappende Kompresse ebenfalls nur noch aufgelegt werden muss und von derselben Klebeschicht gehalten wird. Die Druckstelle 11, die zur Zerstörung des Reservoirs 4 führt kann auf der Schutzschicht markiert sein. Sie kann aber auch weitere Informationen tragen, wie zum Beispiel eine Kennzeichnung des entsprechenden Wirkstoffes.

Das Reservoir kann je nach Wunsch mit einem Desinfektionsmittel, Insektenstichmittel, Brandsalbe oder einem beliebigen Wundbehandlungsmittel versehen sein, wie sie heute unter vielen Markenbezeichnungen erhältlich sind und die noch heute in Flaschen oder Tuben abgepackt in viel zu grossen Quantitäten mitgenommen werden müssen. Die erfindungsgemässen Heftpflaster eignen sich daher ausgezeichnet für Reiseapotheken. Auch die kombinierte Abgabe entsprechend gekennzeichneter Heftpflaster für die häufigsten Anwendungsfälle ist möglich. Hierzu ist es sinnvoll, die bandförmig hergestellten Heftpflaster zu konfektionieren und einzel abzupacken, wie dies bereits heute häufig der Fall ist.

**Ansprüche**

1. Heftpflaster, bestehend aus einer Deckschicht mit selbstklebenden Seitenstreifen, einer ein- oder mehrlagigen Kompresse zur Wundauflage und einer abziehbaren Schutzschicht, dadurch gekennzeichnet, dass zwischen der wundnahen oberen Schicht der Kompresse (5) und der Deckschicht (1) ein Reservoir (4) aus einem für den Durchgang des Wirkstoffes und der den wirkstoffenthaltenden flüssigen oder pastösen Substanz im wesentlichen undurchlässigen Material mit einer Festigkeit, die die Zerstörung des Reservoirs (4) durch Fingerdruck ermöglicht, angebracht ist.

2. Heftpflaster nach Anspruch 1, dadurch gekennzeichnet, dass das Reservoir (4) aus zwei Folien von unterschiedlicher Beschaffenheit besteht, die miteinander unter Freilassung eines Hohlraumes dichtend verbunden sind, wobei die zur oberen Schicht der Kompresse hin gerichtete Folie eine geringere Festigkeit als die zur Deckschicht hin gerichtete Folie aufweist.

3. Heftpflaster nach Anspruch 1, dadurch gekennzeichnet, dass das Reservoir (4) aus zwei Folien gleicher Beschaffenheit gebildet ist, die miteinander unter Freilassung eines Hohlraumes dichtend verbunden sind, wobei die zur oberen Schicht der Kompresse gelegene Folie mindestens eine Sollzerstörungsstelle aufweist.

4. Heftpflaster nach Anspruch 1, dadurch gekennzeichnet, dass das Reservoir (4) zwischen der Deckschicht (1) und der Kompresse (5) angeordnet ist. (Fig. 1)

5. Heftpflaster nach Anspruch 1, dadurch gekennzeichnet, dass das Reservoir (4) zwischen zwei Schichten einer mehrschichtigen Kompresse angeordnet ist. (Fig. 4)

6. Heftpflaster nach Anspruch 3, dadurch gekennzeichnet, dass die Sollzerstörungsstelle (9) durch eine mechanische Schwächung in der Folie geschaffen ist.

7. Heftpflaster nach Anspruch 3, dadurch gekennzeichnet, dass die Sollzerstörungsstelle durch örtliche Bestrahlung der Folie gebildet ist.

8. Heftpflaster nach Anspruch 1, dadurch gekennzeichnet, dass auf der abziehbaren Schutzschicht (7), die zur Zerstörung des Reservoirs (4) geeignete Druckstelle (11) markiert ist.

9. Heftpflaster nach Anspruch 1, dadurch gekennzeichnet, dass auf der abziehbaren Schutzschicht (7) die Wirkstoffe angegeben sind.

FIG.1

FIG.2

FIG.3

FIG.4

FIG.5